# EUROPEAN PATENT SPECIFICATION

(11) **EP 1 771 580 B1**
(45) Date of publication and mention of the grant of the patent: **18.11.2009**
(21) Application number: 05780314.0
(22) Date of filing: 08.07.2005
(51) Int. Cl.: C12Q 1/68

(54) **DIAGNOSTIC AND PROGNOSTIC METHODS OF NON-ALCOHOLIC STEATOHEPATITIS (NASH)**
DIAGNOSE- UND PROGNOSEVERFAHREN DER NICHTALKOHOLISCHEN STEATOHEPATITIS (NASH)
METHODES DIAGNOSTIQUES ET PRONOSTIQUES DE STEATOHEPATITE NON ALCOOLIQUE (NASH)

(30) Priority: 23.07.2004 EP 04103540
(43) Date of publication of application: 11.04.2007
(73) Proprietor: One Way Liver Genomics, S.L., 48160 Derio - Vizcaya (ES)
(72) Inventor: MATO DE LA PAZ, José Marìa Parque Tecnológico de Biz., E-48160 Derio - Vizcaya (ES)
(74) Representative: ABG Patentes, S.L.
(86) International application number: PCT/EP2005/007535
(87) International publication number: WO 2006/008031

(56) References cited:
- ALBERTYN Z.I. ET AL: "SynaProbe - An Ultra High Speed Application for Whole Genome Microarray Probe Design" RESEARCH NEWSLETTER, [Online] May 2004 (2004-05), pages 1-4, XP002301560 Retrieved from the Internet: URL:http://www.synamatix.com/newsletter_ar chive/MTN_May.pdf> [retrieved on 2004-10]
- SREEKUMAR R ET AL: "HEPATIC GENE EXPRESSION IN HISTOLOGICALLY PROGRESSIVE NANALCOHOLIC STEATOHEPATITIS" HEPATOLOGY, WILLIAMS AND WILKINS, BALTIMORE, MD, US, vol. 38, no. 1, July 2003 (2003-07), pages 244-251, XP008036585 ISSN: 0270-9139
- SANTAMARIA E ET AL: "Functional proteomics of nonalcoholic steatohepatitis: Mitochondrial proteins as targets of S-adenosylmethionine" PROCEEDINGS OF THE NATIONAL ACADEMY OF SCIENCES OF USA, NATIONAL ACADEMY OF SCIENCE. WASHINGTON, US, vol. 100, no. 6, 18 March 2003 (2003-03-18), pages 3065-3070, XP002980764 ISSN: 0027-8424
- MARTINEZ-CHANTAR M.L. ET AL: "Spontaneous oxidative stress and liver tumours in mice lacking the methionine adenosyltransferase 1A" THE FASEB JOURNAL, [Online] 7 June 2002 (2002-06-07), pages 1-22, XP002301562 Retrieved from the Internet: URL:http://www.fasebj.org/cgi/reprint/02-0 078fjev1> [retrieved on 2004-10]
- LU S C ET AL: "Methionine adenosyltransferase 1A knockout mice are predisposed to liverinjury and exhibit increased expression of genes involved in proliferation" PROCEEDINGS OF THE NATIONAL ACADEMY OF SCIENCES OF USA, NATIONAL ACADEMY OF SCIENCE, WASHINGTON, DC, US, vol. 98, no. 10, 8 May 2001 (2001-05-08), pages 5560-5565, XP002980762 ISSN: 0027-8424

## Description

### FIELD OF THE INVENTION

The invention relates to early gene markers for non-alcoholic steatohepatitis (NASH). Said genes can be used in methods for diagnosing NASH, more specifically for early diagnosis of NASH, as well as in methods for diagnosing NASH-predisposed liver and for optimizing NASH treatment strategies.

### BACKGROUND OF THE INVENTION

Non-alcoholic steatohepatitis (NASH) is a progressive disease of the liver of unknown etiology characterized histologically by fatty acid accumulation, hepatocyte damage and inflammation resembling alcoholic hepatitis. NASH is a critical stage in the process that spans from hepatic steatosis to cirrhosis and liver failure. A careful history of a lack of significant alcohol intake is essential to establish this diagnostic. NASH is one of the most common causes of elevated aminotransferases in patients referred for evaluation to hepatologists. Obesity and type-2 diabetes are associated to NASH. Since the prevalence of these diseases is increasing, the prevalence of NASH is also expected to increase and therefore, this disease has become an emerging public issue in the United States as well as in other countries.

NASH is thought to arise from the interaction of many different genes and life style factors. Mitochondrial impairment, oxidative stress and metabolic deregulation, have all been involved in the pathogenesis of steatohepatitis. Initial evaluation of patients suspected of NASH when present, are fatigue and right upper abdominal discomfort. Hepatomegaly is found in 90 percent of cases. Ultrasonography is the best method for detection of fatty infiltration of the liver. The principal and often sole laboratory abnormality is an elevation of the serum aminotransferases. Liver biopsy is indicated in symptomatic patients with serum aminotransferase elevations of more than 6 months' duration. The histological features of NASH which are mostly indistinguishable from those of alcoholic hepatitis include macrovesicular fatty infiltration, hepatocellular necrosis and ballooning degeneration, alcoholic hyaline and inflammatory reaction.

As it is true for other complex diseases, the genetic factors contributing to the development of NASH may be more readily identified by combining studies in patients with NASH and in animal models of the disease. One of these models is the *MATIA* knockout (MAT1A-KO) mouse. At about 3 months of age, MATIA-KO livers have normal histology but they are more sensitive to develop severe steatosis. These mice spontaneously develop NASH and hepatocellular carcinoma at about 8 and 15 months of age, respectively [Lu SC, 2001, PNAS USA 98, 5560-5565]. *MATIA* gene encodes for methionine adenosyltransferase I and III, the main enzymes responsible of S-adenosylmethionine (SAMe) synthesis in the liver. Earlier studies concluded that patients with liver cirrhosis and alcoholic hepatitis are deficient in SAMe synthesis; and that treatment with SAMe improves survival in patients with alcoholic liver cirrhosis.

In a previous patent application (WO2004/055520), the inventors developed a method for the diagnosis of NASH by using molecular markers based on the proteomic determination of a set of proteins detected in liver tissue samples, instead of a genomic fingerprint determination, as the method now disclosed in the present invention. None of those genes are included in the present invention.

A method for detecting and quantifying differentially expressed genes in affected individuals at the cirrhotic-stage has been disclosed [Sreekumar R. et al., 2003, Hepatology, July 2003, 244-251]. However, said method cannot be used for diagnosis of NASH, but for diagnosis of cirrhosis.

Early diagnosis of NASH has been held back by the lack of reliable early markers of NAH development. Identification of genes differentially expressed in NASH potentially useful as biological markers or therapeutic targets could lead to the development of new tools for the diagnosis, prognosis and treatment of this disease.

It is difficult to predict from standard clinical and pathological features the clinical course of NASH-predisposed subjects or that of patients suffering NASH. However, it is very important in the treatment of this population to select and implement an appropriate control and therapeutic approaches.

The wide range of NASH predisposed subgroups and variations in disease progression limit the predictive ability of the healthcare professional. The available methods for designing strategies for treating NASH predisposed subjects are complex and time consuming. Treatment in overweight patients consists of gradual moderate weight loss. Hyperglycemia and hyperlipidemia are controlled with medication. A few drugs such as antibiotics, ursodeoxycholic acid, and vitamin E are under evaluation. However, despite the improvement in liver tests, fatty infiltration detected by ultrasonography remained unchanged. Liver transplantation is indicated for patients who have developed advanced cirrhosis.

Therefore, there is a need for the diagnosis, specially for early diagnosis, prognosis and treatment of NASH.

### SUMMARY OF THE INVENTION

Data generated from genome-wide expression profiling (microarray data) of liver samples from patients with NASH and from a mouse model of NASH (MATIA-KO) in order to obtain the genomic signature of NASH have been analyzed. An analysis technique that combines bioinformatics and statistical methods has been used. This genomic signature of NASH accurately distinguishes between patients with early-stage NASH and healthy controls.

An aspect of the invention relates to a method for *in vitro* diagnosing non-alcoholic steatohepatitis (NASH) in liver tissue samples, based in the co-expression of a set of 85 genes which shows specific differences in gene expression between patients and controls. The method allows the easy and reliable evaluation of the potential risk of a subject to develop NASH, meaning that it allows the determination of this subject, within a group of population, for the risk of developing NASH. The mentioned subject may be a subject who has not been previously diagnosed with NASH or a subject who has been diagnosed with NASH to be confirmed. As an example, a subject who has not been previously diagnosed with NASH or who has no symptoms may be analyzed in order to obtain information about the possibility of that subject of developing NASH in the future. Therefore, in a particular embodiment, the method allows the evaluation of the predisposition (early diagnosis) of a subject to develop NASH, while in another particular embodiment, the same method allows the confirmation diagnosis of the existence of NASH in a subject.

In other aspect, the invention relates to a method for *in vitro* determining the predisposition of a subject to develop NASH, or for determining the stage or severity of NASH in a subject, or for monitoring the effect of the therapy administered to said subject with said condition.

In other aspect, the disclosure relates to a solid-phase nucleic acid array. The array comprises a set of probes useful for detecting and identifying the NASH early gene marker set, that consists essentially of all of the genes shown in Tables 2 and 3, fixed to a solid substrate.

A kit comprising a set of probes useful for detecting and identifying the NASH early gene marker set, optionally fixed on a solid substrate, constitutes another aspect of the present disclosure.

A further aspect of the present invention relates to an *in vitro* method for identifying and/or evaluating the efficacy of a potentially therapeutic agent against NASH.

### BRIEF DESCRIPTION OF THE DRAWINGS

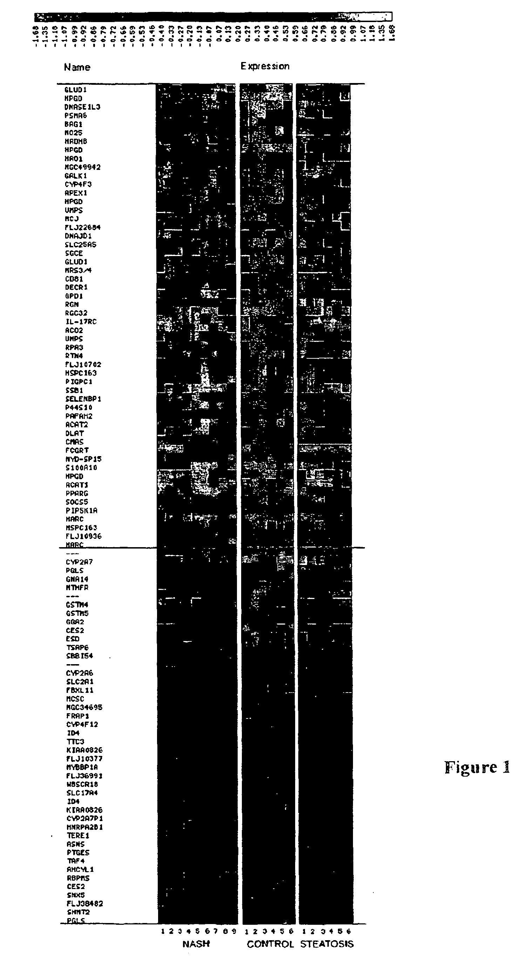

Figure 1 shows the liver expression of the 98 Affymetrix human probes, corresponding to the 85 validated early gene markers of NASH, in NASH patients, healthy controls and patients with hepatic steatosis. Pseudo colors indicate differential expression (red, up-regulation; green, down-regulation; black, no expression change). Shown values are the difference between expression levels and mean expression level measured in standard deviations. Name, gene name; NASH, patients with non-alcoholic steatohepatitis; Control, healthy controls; Steatosis, patients with hepatic steatosis.

### DETAILED DESCRIPTION OF THE INVENTION

### Definitions

In order to facilitate the comprehension of the present patent application we list below the meaning of some terms and expressions within the context of the invention.

The term "subject", as used herein, refers to all animals classified as mammals and includes, but is not restricted to, domestic and farm animals, primates and humans, e.g., human beings, non-human primates, cows, horses, pigs, sheeps, goats, dogs, cats, or rodents. Preferably, the subject is a male or female human of any age or race. Illustrative, non limitative examples of subjects according to the invention include humans either suspected of having fatty infiltration of the liver, or patients with serum aminotransferase elevations for a long period (over 6 months' duration), or patients suspected of having NASH. Methods for identifying subjects suspected of having NASH may include physical examination, subject's family medical history, subject's medical history, liver biopsy, or a number of imaging technologies such as ultrasonography.

The term "NASH" refers to non-alcoholic steatohepatitis. Diagnostic methods for NASH and the clinical delineation of NASH diagnoses are well known to those of skill in the medical arts.

The expression "early gene markers for NASH" refers to genes that by their overexpression (up-regulation) or repression (down-regulation) indicate NASH at early stages of said pathology or the predisposition of a subject in time of suffering NASH. According to the invention, the early gene markers for NASH comprise the set of 85 genes listed in Tables 2 and 3 which further constitute the genomic signature (fingerprint) of (early) NASH.

The term "gene" refers to a sequence of nucleotides in a genetic nucleic acid which codes for a polypeptide and variants thereof and may represent a portion of the coding sequence, or the complete coding sequence or a portion of a non-coding sequence.

### Genomic signature of NASH in humans

The invention described herein relates to the identification of a set of genes expressed in NASH-predisposed liver tissue that are predictive of the clinical outcome of the liver pathology. Changes in cell phenotype in NASH are often the result of one or more changes in the genome expression of the cell. Some genes are expressed in different stages of NASH, which have different prognoses and require different treatment regimens to optimize patient outcome. The differential expression of NASH-associated genes can be examined, for example, by the assessment of nucleic acid expression in a liver tissue sample.

The instant invention is based on the identification of a set of 85 differentially expressed genes in NASH-predisposed liver tissue samples compared to liver tissue samples from control subjects (e.g., NASH free subjects). Table I shows the set of 85 discriminative (early) gene markers specific of NASH in human liver tissue samples constituting the genomic signature (fingerprint) of (early) NASH in humans.

**Table 1**

| **Genomic signature of NASH in humans** | | |
|---|---|---|
| **NAME** | **DESCRIPTION** | **EXPRESSION IN HUMANS WITH NASH** |
| ***Apoptosis regulador*** | | |
| BAG 1 | Bcl2-associated athanogene I | + |
| ***Chaperone*** | | |
| BAG1 | Bc12-associated athanogene 1 | + |
| WBSCR18 | Syntaxin 1A (brain) | + |
| ***Defense*/*****immunity protein**_Blood coagulation factor* | | |
| F11 | Coagulation factor XI | - |
| ***Enzyme**_Hydrolase* | | |
| MGC34695 | Clone:1300019N10, hypothetical protein | - |
| NYD-SP15 | cytidine and dCMP deaminase domain containing 1 (NYD-SP15) | + |
| AHCYL1 | S-adenosylhomocysteine hydrolase-like 1 | - |
| PAFAH2 | Similar to platelet-activating factor acetylhydrolase 2, + 40kDa (PAFAH2) | + |
| APEX I | Apurinicapyrimidinic endonuclease | + |
| CES2 | Carboxylesterase 2 (CES2) | - |
| DNASEIL3 | Deoxyribonuclease I-like 3 | + |
| ESD | Esterase 10 | - |
| F11 | Coagulation factor XI | - |
| GNA 14 | Guanine nucleotide binding protein, alpha 14 | - |
| PGLS | 6-phosphogluconolactonase | - |
| PSMA6 | Proteasome (prosome, macropain) subunit, alpha type 6 | + |
| ***Enzyme**_Isomerase* | | |
| HPGD | Hydroxyprostaglandin dehydrogenase 15-(NAD) | + |
| PTGES | Prostaglandin E synthase | - |
| ***Enzyme**_Kinase* | | |
| NYD-SP15 Cytidine and dCMP deaminase domain containing I NYD-SP15 (NYD-SP15) | | |
| | | + |
| FRAP1 | FK506 binding protein 12-rapamycin associated protein 1 | - |
| GALK1 | Galactokinase | + |
| PIP5K1B | Phosphatidylinositol-4-phosphate 5-kinase, type 1 beta | + |
| ***Enzyme**_Ligase* | | |
| ASNS | Asparagine synthetase | - |
| ***Enzyme**_Lyase* | | |
| ACO2 | Aconitase 2, mitochondrial | + |
| APEX I | Apurinicapyrimidinic endonuclease | + |
| UMPS | Uridine monophosphate synthetase | + |
| ***Enzyme**_Monooxygenase* | | |
| CYP2A6 | Cytochrome P450, 2 A6 | |
| CY4F12 | Cytochrome P450, F12 | |
| CYP2A7 | Cytochrome P450, 2A7 | - |
| CYP2A7P I | Cytochrome P450, 2A7 | |
| CYP4F3 | Cytochrome P450, subfamily IVF, polypeptide 3 (leukotriene B4 omega hydroxylase) | + |
| ***Enzyme**_Oxidoreductase* | | |
| CYP2A7 | Cytochrome P450, 2 A7 | - |
| CYP2A7P1 | Cytochrome P450, 2 A7 | |
| CYP4F3 | Cytochrome P450, subfamily IVF, polypeptide 3 | + |
| CYP2A7P1 | (leukotriene B4 omega hydroxylase) | |
| DECR1 | 2,4-dienoyl CoA reductase 1, mitochondrial | + |
| GPD1 | Glycerol phosphate dehydrogenase 1, cytoplasmic adult | + |
| HAO1 | Hydroxyacid oxidase 1, liver | + |
| HPGD | Hydroxyprostaglandin dehydrogenase 15-(NAD) | + |
| MTHFR | 5,10-methylenetetrahydrofolate reductase (NADPH) | - |
| ***Enzyme**_Transferase* | | |
| | Hydroxyacyl-Coenzyme A dehydrogenase/3-ketoacyl- | |
| HADHB | Coenzyme A thiolase/enoyl-Coenzyme A hydratase, beta subunit (HADHB) | + |
| ACAT1 | Acetyl-Co A acetyltransferase 1 | + |
| ACAT2 | Acetyl CoA transferase-like protein | + |
| CMAS DLAT | CMP-N-acetylneuraminic acid synthetase Dihydrolipoamide S-acetyltransferase (E2 component of | + |
| | pyruvate dehydrogenase complex) | |
| FRAP1 | FK506 binding protein 12-rapamycin associated protein I | - |
| GALK1 | Galactokinase | + |
| GSTM5 | Glutathione S-transferase, mu 5 | - |
| GSTM4 | Glutathione transferase GSTM4 | - |
| PIP5K1B | Phosphatidylinositol-4-phosphate 5-kinase, type I beta | + |
| SHMT2 | Serine hydroxymethyltransferase 2 (mitochondrial) | - |
| UMPS | Uridine monophosphate synthetase | + |
| ***Enzyme regulator**_ Enzyme activator* | | |
| RGC32 | Response gene to complement 32 (RGC32) | + |
| ***Enzyme regulator**_ Kinase regulator* | | |
| RGC32 | Response gene to complement 32 (RGC32) | + |
| ***Ligand binding or carrier*** | | |
| MRS3/4 | Putative mitochondrial solute carrier (MRS3/4) | + |
| SLC25A5 | Solute carrier family 25 (mitochondrial carrier; adenine | + |
| | nucleotide translocator) | |
| ***Ligand binding or carrier**_Calcium binding* | | |
| MCSC | Mitochondrial Ca2+-dependent solute carrier (MCSC) | - |
| MO25 | Calcium binding protein, 39 | + |
| RGN | Regucalcin | + |
| S100A10 | S100 calcium binding protein A10 (calpactin) | + |
| ***Ligand binding or carrier**_Heavy metal binding* | | |
| NYD-SP15 | Cytidine and dCMP deaminase domain containing 1 (NYD-SP15) | + |
| ACO2 | Aconitase 2, mitochondrial | + |
| ***Ligand binding or carrier**_Nucleic acid binding* | | |
| FLJ10377 | Expressed sequence A1503051 | - |
| FLJ36991 | Zinc finger protein 565 | - |
| APEX1 | Apurinicapyrimidinic endonuclease | + |
| DNASEIL3 | Deoxyribonuclease 1-like 3 | + |
| TAF4 | TAF4A RNA polymerase II, TATA box binding protein | - |
| | (TBP)-associated factor 135 kDa (TAF4) | |
| PPARG | Peroxisome proliferator activated receptor gamma | + |
| RBPMS | RNA binding protein gene with multiple splicing | + |
| ***Ligand binding or carrier**_Nucleolide binding* | | |
| FLJ10702 | ADP-ribosylation factor-like 10C | + |
| GALK1 | Galactokinase | + |
| GNA14 | Guanine nucleotide binding protein, alpha 14 | - |
| GPD1 | Glycerol phosphate dehydrogenase I, cytoplasmic adult | + |
| SLC2A1 | Solute carrier family 2 (facilitated glucose transporter), | - |
| | member I | |
| ***Ligand binding or carrier**_Protein binding* | | |
| RGC32 | Response gene to complement 32 (RGC32) | + |
| BAG1 | Bcl2-associated athanogene I | + |
| HARC | Cell division cycle 37 homolog (S. cerevisiae)-like | + |
| FCG RT | Fc receptor, lgG, alpha chain transporter | + |
| ***Ligand binding or carrier**_ Transcription factor* | | |
| TAF4 | TAF4A RNA polymerase II, TATA box binding protein | - |
| | (TBP)-associated factor 135 kDa (TAF4) | |
| PPARG | Peroxisome proliferator activated receptor gamma | + |
| ***Signal transducer*** | | |
| GNA14 | Guanine nucleotide binding protein, alpha 14 | - |
| ***Signal transducer**_ Receptor* | | |
| FLJ22684 | RIKEN cDNA 5031409J19 gene | + |
| FCGRT | Fc receptor, IgG, alpha chain transporter | + |
| PPARG | Peroxisome proliferator activated receptor gamma | + |
| ***Translation regulador*** | | |
| PPARG | Peroxisome proliferator activated receptor gamma | + |
| ***Transporter*** | | |
| SLC 17A4 | Solute carrier family 17 (sodium phosphate), member 2 | - |
| SLC25A5 | Solute carrier family 25 (mitochondrial carrier; adenine nucleotide translocator) | + |
| ***Transporter**_ Carbohydrate transporter* | | |
| SLC2A1 | Solute carrier family 2 (facilitated glucose transporter), | - |
| | member 1 | |
| ***Transporter**_Carrier* | | |
| SLC2A1 | Solute carrier family 2 (facilitated glucose transporter), | - |
| | member 1 | |
| ***Transporter**_Electron transporter* | | |
| HPGD | Hydroxyprostaglandin dehydrogenase 15-(NAD) | + |
| HAOI | Hydroxyacid oxidase 1, liver | + |
| ***Transporter**_Protein transporter* | | |
| SNX5 | Sorting nexin 5 | - |

| *Unkown molecular function* | | |
|---|---|---|
| MGC49942 | Myb-like DNA-binding domain containing protein | + |
| SBB154 | Hypothetical transmembrane protein SBB154 | - |
| SOCS5 | Socs-5 | + |
| KIAA0826 | RIKEN cDNA 2510002A14 gene | - |
| P44S10 | Proteasome regulatory particle subunit P44S10 | + |
| FLJ10936 | Transmembrane protein 19 (FLJ10936) | + |
| FLJ38482 | RIKEN cDNA 3110005G23 gene, MGC:28055 | - |
| SSB1 | SPRY domain-containing SOCS box protein SSB-1 | + |
| HNRPA2B1 | Heterogeneous nuclear ribonucleoprotein A2B1 (HNRPA2B1) | + |
| GLUD1 | Glutamate dehydrogenase I (GLUD1) | + |
| CD81 | CD 81 antigen | + |
| HSPC163 | HSPC163 protein | + |
| DNAJD1 | DnaJ (Hsp40) homolog, subfamily D, member I | + |
| Fbx111 | F-box protein FBL11 | - |
| GGA2 | Homolog to ADP-ribosylation factor binding protein | - |
| | GGA2 (Gamma-adaptin related protein 2) | |
| ID4 | Inhibitor of DNA binding 4 (ID4) | - |
| 1L-17RC | Interleukin 17 receptor-like | + |
| MYBBP1A | MYB binding protein (P160) 1a | - |
| PERP | P53 apoptosis effector related to Pmp22 (PIGPC1) | + |
| RPA3 | Replication protein A3, 14kDa | + |
| RTN4 | GLUT4 VESICLE 20 KDA PROTEIN | + |
| SELENBP1 | Selenium binding protein in chromosome 14 open reading | + |
| | frame 52 | |
| SGCE | Sarcoglycan, epsilon | + |
| TERE1 | Transitional epithelia response protein | - |
| TSAP6 | Dudulin 2 (TSAP) | - |
| TTC3 | Tetratricopeptide repeat domain | - |

Among the genes comprising the genomic signature of NASH, there are 34, more than would be expected by chance, which are enzymes, the majority of them being hydrolases, transferases, and oxidoreductases. The list in Table 1 also includes several ligand-binding genes (heavy metal binding, nucleotide binding, protein binding, receptors and transcription factors); transporters (carbohydrate, electron transporter, and protein transporters); apoptosis regulators; chaperones; blood coagulation factors; and several genes with unknown function. However, all the genes comprised in the cluster constituting the genomic signature of NASH, even those with unknown function, have value as integrated diagnosis tool for the disease, by measuring the co-expression of the genes comprised in the cluster, in patients as compared with the reference gene co-expression in control samples.

The genomic signature of NASH provided by the instant invention accurately distinguishes between patients with early-stage NASH and healthy controls. Patients with liver steatosis have a genomic signature intermediate between that of patients with NASH and controls, offering the prospect of early diagnosis, which improves the chance to intervene with an effective treatment.

The genes identified in the genomic signature of NASH permit, *inter alia,* a rapid screening of pathological samples by conventional techniques, such as, for example, by nucleic acid microarray hybridization technology, in order to determine the expression of the specific genes and thereby to predict the outcome of NASH. Such screening is beneficial, for example, in selecting the course of treatment to provide to the patient, and to monitor the efficacy of a treatment already administered to a patient. Accordingly, the evaluation and comparison of said levels of gene expression in liver tissue samples can be both, diagnostic or prognostic of NASH. For example, an elevated level of expression of the genes described in Table 2 (below) together with a decreased level of expression of the genes described in Table 3 (below), in a liver tissue sample, is indicative of NASH, or is indicative of risk or predisposition of the subject to develop NASH. Therefore, the above mentioned finding can be used, for example, in any of the following methods: diagnostic assays, prognostic assays, monitoring treatment, monitoring clinical trials and screening assays as further described herein.

The invention simplifies prognosis determination by providing an identified set of early genes whose expression in predisposed NASH subjects or diagnosed NASH subjects, predicts clinical outcome as defined by NASH, cirrhosis, hepatocellular carcinoma, or death.

The expression gene set has multifold uses including, but not limited to, the following examples. The expression gene set may be used as a prognostic tool for NASH-predisposed patients, to make possible more finely tuned diagnosis of NASH and allow healthcare professionals to tailor treatment to individual patients' needs. The invention can also assess the efficacy of NASH treatment by determining progression or regression of NASH in patients before, during, and after NASH treatment. Another utility of the expression gene set is in the biotechnology and pharmaceutical industries' research on disease pathway discovery for therapeutic targeting. The invention can identify alterations in gene expression in NASH and can also be used to uncover and test candidate pharmaceutical agents to treat NASH. The invention also presents potential targets for drug development because it identifies genes that are differentially expressed in outcome NASH liver tissue, which can be utilized in the development of drugs to treat such pathology, e.g., by reducing the expression of the genes (Table 2) or by reducing the activity of proteins encoded by said genes, or, alternatively, by increasing the expression of the genes (Table 3) or by increasing the activity of proteins encoded by said genes.

The genomic signature of NASH in humans was obtained by analysis of the data generated from genome-wide expression profiling (microarray data) of liver samples from patients with NASH and from a mouse model of NASH (MAT1A-KO) by means of an analysis technique that combines bioinformatics and statistical methods (Example 1).

### Diagnostic and prognostic methods

In an aspect, the invention relates to an *in vitro* method for diagnosing NASH in a subject, or for early diagnosing NASH in a subject, or for determining the predisposition of a subject to develop NASH, or for determining the stage or severity of NASH in a subject, or for monitoring the effect of the therapy administered to said subject with said condition, hereinafter referred to as the method of the invention, which comprises:
a) quantifying the expression level of the set of 85 genes, shown in Table 2 and Table 3, in a liver tissue sample from said subject, and
b) comparing the levels of said genes to the levels of the same genes of a control sample;
wherein an increase in the expression levels of the genes of Table 2 and a decrease in the expression levels of the genes of Table 3, relative to the levels of the same genes of the control sample, is indicative of NASH, i.e., it is an indication that said subject is suffering from NASH, or indicative of a predisposition of the subject to develop NASH.

In order to carry out the method of the invention, a sample is obtained from the subject under study. In a particular embodiment, the sample is a liver tissue sample, which can be obtained by conventional methods, e.g., biopsy, by using methods well known to those of ordinary skill in the related medical arts. Methods for obtaining the sample from the biopsy include gross apportioning of a mass, or microdissection or other art-known cell-separation methods. Samples can be obtained from subjects previously diagnosed or not with NASH, or from subjects who are receiving or have previously received anti-NASH treatment. In an embodiment, the sample is a sample from a subject having normal hepatic function tissue, i.e., a tissue determined, by one of ordinary skill in the medical art, to have no evidence of fatty infiltration of the liver, often accompanied by hepatocellular damage with inflammation.

Because of the variability of the cell types in diseased-tissue biopsy material, and the variability in sensitivity of the diagnostic methods used, the sample size required for analysis may range from 1, 10, 50, 100, 200, 300, 500, 1,000, 5,000, 10,000, to 50,000 or more cells. The appropriate sample size may be determined based on the cellular composition and condition of the biopsy, and the standard preparative steps for this determination and subsequent isolation of the nucleic acid for use in the invention are well known to one of ordinary skill in the art. An example of this, although not intended to be limiting, is that in some instances a sample from the biopsy may be sufficient for assessment of RNA expression without amplification, but in other instances the lack of suitable cells in a small biopsy region may require use of RNA conversion and/or amplification methods or other methods to enhance resolution of the nucleic acid molecules. Such methods, which allow the use of limited biopsy materials, are well known to those of ordinary skill in the art and include, but are not limited to: direct RNA amplification, reverse transcription of RNA to cDNA, amplification of cDNA, or the generation of radio-labelled nucleic acids.

Tables 2 and 3 identify the set of 85 genes which accurately distinguish between patients with early-stage NASH and healthy controls. Said genes were identified by analyzing the data generated from genome-wide expression profiling of liver samples from patients with NASH and from MAT1A-KO mice in order to obtain the genomic signature of NASH in humans (Example 1).

**Table 2**

| **NASH up-regulated genes** | |
|---|---|
| **NAME** | **DESCRIPTION** |
| ***PAFAH2*** | Similar to platelet-activating factor acetylhydrolase 2, 40kDa (PAFAH2) |
| ***APEX1*** | Apurinicapyrimidinic endonucleaseumps |
| ***DNASEIL3*** | Deoxyribonuclease I-like 3 |
| ***PSMA6*** | Proteasome (prosome, macropain) subunit, alpha type 6 |
| ***HPGD*** | Hydroxyprostaglandin dehydrogenase 15-(NAD) |
| ***ACO2*** | Aconitase 2, mitochondrial |
| ***UMPS CYP4F3*** | Uridine monophosphate synthetase Cytochrome P450, subfamily IVF, polypeptide 3 (leukotriene |
| | B4 omega hydroxylase) |
| ***DECR1*** | 2,4-dienoyl CoA reductase 1, mitochondrial |
| ***GPD1*** | Glycerol phosphate dehydrogenase 1, cytoplasmic adult Hydroxyacyl-Coenzyme A dehydrogenase/3-ketoacyl- |
| ***HADHB*** | Coenzyme A thiolase/enoyl-Coenzyme A hydratase, beta subunit (HADHB) |
| ***ACAT1*** | Acetyl-Co A acetyltransferase 1 |
| ***ACAT2*** | Acetyl CoA transferase-like protein |
| ***CMAS*** | CMP-N-acetylneuraminic acid synthetase |
| ***DLAT*** | Dihydrolipoamide S-acetyltransferase (E2 component of pyruvate dehydrogenase complex) |
| ***P1P5K1B*** | Phosphatidylinositol-4-phosphate 5-kinase, type I beta |
| ***UMPS*** | Uridine monophosphate synthetase |
| ***RGC32*** | Response gene to complement 32 (RGC32) |
| ***MRS3*/*4*** | Putative mitochondrial solute carrier (MRS3/4) |
| ***SLC25A5*** | Solute carrier family 25 (mitochondrial carrier; adenine nucleotide translocator) |
| ***MO25*** | Calcium binding protein, 39 |
| ***RGN*** | Regucalcin |
| ***S100A10*** | S100 calcium binding protein A10 (calpactin) |
| ***NYD-SP15*** | Cytidine and dCMP deaminase domain containing 1 (NYD-SP15) |
| ***PPARG*** | Peroxisome proliferator activated receptor gamma |
| ***FLJ10702*** | ADP-ribosylation factor-like 10C |
| ***GALK1*** | Galactokinase |
| ***BAG1*** | Bc12-associated athanogene 1 |
| *HARC* | Cell division cycle 37 homolog (S. cerevisiae)-like |
| ***FCGRT*** | Fc receptor, IgG, alpha chain transporter |
| ***FLJ22684*** | RIKEN cDNA 5031409J19 gene |
| ***HPGD*** | Hydroxyprostaglandin dehydrogenase 15-(NAD) |
| *HAO1* | Hydroxyacid oxidase 1, liver |
| ***MGC49942*** | Myb-like DNA-binding domain containing protein |
| ***SOCS5*** | Socs-5 |
| ***P44S10*** | Proteasome regulatory particle subunit P44S10 |
| ***FLJ10936*** | Transmembrane protein 19 (FLJ 10936) |
| ***SSB1*** | SPRY domain-containing SOCS box protein SSB-1 |
| ***GLUD1*** | Glutamate dehydrogenase I (GLUD1) |
| ***CD81*** | CD 81 antigen |
| ***HSPC163*** | HSPC163 protein |
| ***DNAJDI*** | DnaJ (Hsp40) homolog, subfamily D, member 1 |
| ***lL-17RC*** | Interleukin 17 receptor-like |
| ***PERP*** | P53 apoptosis effector related to Pmp22 (PIGPC1) |
| ***RPA3*** | Replication protein A3, 14kDa |
| ***RTN4*** | GLUT4 VESICLE 20 KDA PROTEIN |
| ***SELENBP1*** | Chromosome 14 open reading frame 52 |
| ***HNRPA2B1*** | Heterogeneous nuclear ribonucleoprotein A2B1 (HNRPA2B1) |
| ***SGCE*** | Sarcoglycan, epsilon |

**Table 3**

| **NASH down-regulated genes** | |
|---|---|
| **NAME** | **DESCRIPTION** |
| ***WBSCR18*** | Syntaxin 1A (brain) |
| ***F11*** | Coagulation factor XI |
| ***RBPMS*** | RNA binding protein gene with multiple splicing |
| ***MGC34695*** | Clone:1300019N 10, hypothetical protein |
| ***AHCYL1*** | S-adenosylhomocysteine hydrolase-like 1 |
| ***CES2*** | Carboxylesterase 2 (CES2) |
| ***ESD*** | Esterase 10 |
| ***GNA14*** | Guanine nucleotide binding protein, alpha 14 |
| ***PGLS*** | 6-phosphogluconolactonase |
| ***PTGES*** | Prostaglandin E synthase |
| ***FRAP1*** | FK506 binding protein 12-rapamycin associated protein 1 |
| ***ASNS*** | Asparagine synthetase |
| ***CYP2A6*** | Cytochrome P450, 2 A6 |
| ***CY4PF12*** | Cytochrome P450, F12 |
| ***CYP2A7*** | Cytochrome P450, 2 A7 |
| ***CYP2A7P1*** | Cytochrome P450, 2 A7 |
| ***MTHFR*** | 5,10-methylenetetrahydrofolate reductase (NADPH) |
| ***GSTM5*** | Glutathione S-transferase, mu 5 |
| ***GSTM4*** | Glutathione transferase GSTM4 |
| ***SHMT2*** | Serine hydroxymethyltransferase 2 (mitochondrial) |
| ***MCSC*** | Mitochondrial Ca2+-dependent solute carrier (MCSC) |
| ***FLJ10377*** | Expressed sequence A1503051 |
| ***FLJ36991*** | Zinc finger protein 565 |
| ***TAF4*** | TAF4A RNA polymerase II, TATA box binding protein (TBP)-associated factor 135 kDa (TAF4) |
| ***SLC17A4*** | Solute carrier family 17 (sodium phosphate), member 2 |
| | Solute carrier family 2 (facilitated glucose transporter), member 1 |
| ***SNX5*** | Sorting nexin 5 |
| ***SBBI54*** | Hypothetical transmembrane protein SBBI54 |
| ***KIAA0826*** | RIKEN cDNA 2510002A14 gene |
| ***FLJ38482*** | RIKEN cDNA 3110005G23 gene, MGC:28055 |
| ***FbxlII*** | F-box protein FBL11 |
| ***GGA2*** | Homolog to ADP-ribosylation factor binding protein GGA2 (Gamma-adaptin related protein 2) |
| ***ID4*** | Inhibitor of DNA binding 4 (ID4) |
| ***MYBBP1A*** | MYB binding protein (P160) la |
| ***TERE1*** | Transitional epithelia response protein |
| ***TSAP6*** | Dudulin 2 (TSAP) |
| ***TTC3*** | Tetratricopeptide repeat domain |

Quantification of the expression level of the set of 85 genes shown in Tables 2 and 3, in a liver tissue sample, can be carried out by virtually any technique suitable for detecting and quantifying a plurality of genes. An approach for quantifying, in a liver tissue sample, the expression level of the set of 85 genes shown in Tables 2 and 3 comprises identifying and quantifying the corresponding RNA transcripts in the liver tissue sample by analysis of gene expression in said liver tissue sample. Gene expression analysis can be carried out using nucleic acid arrays based on hybridization methods.

Hybridization methods for nucleic acids are well known to those of ordinary skill in the art (see, e.g. Molecular Cloning: A Laboratory Manual, J. Sambrook, et al., eds., Second Edition, Cold Spring Harbor Laboratory Press, Cold Spring Harbor, N.Y., 1989, or Current Protocols in Molecular Biology, F. M. Ausubel, et al., eds., John Wiley & Sons, Inc., New York). The nucleic acid molecules from a liver tissue sample hybridize under stringent conditions to nucleic acid markers expressed in NASH liver, said markers being the set of genes shown in Tables 2 and 3.

In a particular embodiment, the quantification of the expression levels, of said set of 85 genes shown in Tables 2 and 3 is carried out by nucleic acid microarray hybridization technology. According to the present invention, standard hybridization techniques of microarray technology are used to assess patterns of gene expression and identify gene marker expression. Microarray technology, which is also known by other names including: DNA chip technology, gene chip technology, and solid-phase nucleic acid array technology, is well known to those of ordinary skill in the art and is based on, but not limited to, obtaining an array of identified gene probes fixed on a solid substrate, labelling target molecules with reporter molecules (e.g., radioactive, chemiluminescent, or fluorescent tags such as fluorescein, Cye3-dUTP, or Cye5-dUTP), hybridizing target genes to the probes, and evaluating target-probe hybridization. A probe with a nucleic acid sequence that perfectly matches the target sequence will, in general, result in detection of a stronger reporter-molecule signal than will probes with less perfect matches. Many components and techniques utilized in nucleic acid microarray technology are presented in The Chipping Forecast, Nature Genetics, Vol.21, January 1999.

According to the invention, probes are selected from the group of nucleic acids including, but not limited to, DNA, genomic DNA (gDNA), cDNA and oligonucleotides; and may be natural or synthetic. Oligonucleotide probes preferably are 20 to 25-mer oligonucleotides whereas DNA/cDNA probes preferably are 500 to 5,000 bases in length; nevertheless, in both cases, other lengths may be used.

Virtually any probe capable of detecting or identifying any of the genes listed in Table I can be used for working the invention. The nucleotide sequences of the 85 genes listed in Table 1 are known and/or may be obtained from suitable databases, such as, for example, the International Nucleotide Sequence Database Collection, a collection which includes DNA Database Bank of Japan (DDBJ), European Molecular Biology Laboratory (EMBL/EBI) and Nucleotide Sequence Database (GenBank, USA) [http://www.ncbi.nim.nih.gov/collab]. Therefore, the person skilled in the art can design the probes to be used for identifying and quantifying the 85 genes listed in Table 1. Available software can be used by those skilled in the art in order to achieve that aim. Usually, said probes will be labelled for direct or indirect detection. In a particular embodiment, probes are labelled enzymatically with a radioactive isotope or with a fluorescent marker for direct detection, whereas in another particular embodiment, probes are chemically or enzymatically labelled for indirect detection by, for example, luminescent or fluorimetric techniques.

According to the present invention, microarray substrates may include but are not limited to glass, silica, aluminosilicates, borosilicates, metal oxides such as alumina and nickel oxide, various clays, nitrocellulose, or nylon, preferably, the substrate is a glass substrate.

The method of the invention also includes the step of comparing the levels of gene expression of the genes listed in Tables 2 and 3 quantified in a liver tissue sample from the subject under study to the levels of gene expression of the same genes in a control sample (i.e., baseline level or reference value). An increase in the expression levels of the genes of Table 2 together with a decrease in the expression levels of the genes of Table 3, relative to the levels of the same genes of the control sample, is indicative of NASH, or indicative of a predisposition of the subject to develop NASH.

In an embodiment, the control sample is a liver tissue sample from NASH free subjects, i.e., from a subject having normal hepatic function tissue (i.e., control subjects with respect to NASH). This is particularly useful for early diagnosing NASH and/or for determining the predisposition of a subject to develop NASH.

In another embodiment, the control sample is a liver tissue sample from a subject previously diagnosed with NASH, or from subjects who are receiving or have previously received anti-NASH treatment, what is particularly useful for determining the stage or severity of NASH in a subject, or for monitoring the effect of the therapy administered to said subject with said condition, thus providing the physician with an important tool for treating NASH.

The method of the invention, based on the measurement of the levels of gene expression of a set (plurality) of genes in liver tissue samples is highly sensitive and specific. Said method differs from traditional NASH diagnostic and classification techniques with respect to the speed, simplicity, and reproducibility of the NASH diagnostic histology. The invention, thus, simplifies prognosis determination by providing an identified set of early genes whose expression, in predisposed NASH subjects or diagnosed NASH subjects, predicts clinical outcome as defined by NASH, cirrhosis, hepatocellular carcinoma, or death.

In other aspect, the invention relates to the use of the set of 85 genes, shown in Tables 2 and 3, i.e., the genomic signature of NASH, for *in vitro* diagnosing NASH in a subject, or for early diagnosing NASH in a subject, or for determining the predisposition of a subject to develop NASH, or for determining the stage or severity of NASH in a subject, or for monitoring the effect of the therapy administered to said subject with said condition. In a particular embodiment, the expression levels of said set of 85 genes shown in Tables 2 and 3 is quantified by solid-phase nucleic acid array hybridization technology.

In other aspect, the disclosure relates to a solid-phase nucleic acid array. The array comprises a set of probes useful for detecting and identifying the NASH early gene marker set, that consists essentially of all of the genes shown in Tables 2 and 3 (or in Table 1), fixed to a solid substrate. In a particular embodiment, the array provided by the instant invention consists of the set of probes useful for detecting and identifying the NASH early gene marker set, i.e., all the genes shown in Tables 2 and 3, together with some positive and/or negative hybridization controls. Illustrative, non-limitative, examples of said hybridization controls include one or more probes useful for detecting and identifying one or more target genes which should be expressed [said target genes being genes not included among the genes constituting the genomic signature of NASH according to this invention, i.e., being genes other than those included in Tables 2 and 3, for example, constitutively expressed genes such as ribosome sub-unit 18S, etc.] and, consequently, a hybridization product is expected between said probe(s) and said target gene(s) (positive hybridization controls) as well as one or more probes useful for detecting and identifying one or more genes which should not be expressed under the analysed conditions [i.e., genes which are known not being expressed under NASH condition (said genes are known from the assays which rendered the genomic signature of NASH), or even genes which are not present in a mammal tissue sample, e.g., a *Drosophila melanogaster* gene having no orthologous gene in mammals, etc.)] and, consequently, a hybridization product is not expected between said probe(s) and said gene(s) (negative hybridization controls).

In a particular embodiment, probes are selected from the group of nucleic acids including, but not limited to, DNA, gDNA, cDNA and oligonucleotides; and they may be of natural or synthetic origin. Oligonucleotide probes preferably are 20 to 25-mer oligonucleotides whereas DNA/cDNA probes preferably are 500 to 5,000 bases in length; nevertheless, in both cases, other lengths may be used. Virtually any probe capable of detecting or identifying any of said genes can be used in the manufacture of the array provided by the instant disclosure. As mentioned above, the nucleotide sequences of the 85 genes listed in Table I are known and/or may be obtained from suitable databases, such as, those previously mentioned. Therefore, the person skilled in the art can design the probes to be used for identifying and quantifying the 85 genes listed in Table 1. Available software can be used by those skilled in the art in order to achieve that aim. Usually, said probes will be labelled for direct or indirect detection. In a particular embodiment, probes are labelled enzymatically with a radioactive isotope or with a fluorescent marker for direct detection, whereas in another particular embodiment, probes are chemically or enzymatically labelled for indirect detection by, for example, luminescent or fluorimetric techniques.

Also, virtually any suitable solid substrate can be used in the manufacture of the array provided by the instant disclosure. Illustrative, non-limitative examples of solid substrates which can be used include glass, silica, aluminosilicates, borosilicates, metal oxides such as alumina and nickel oxide, clay, nitrocellulose, or nylon, preferably, glass.

The probes can be fixed (immobilized) to the solid substrate by any conventional technique, for example, by non-covalent immobilization techniques (e.g., by electronic immobilization), or by immobilization techniques based on the covalent bond of the probes to the solid substrate by chemical means such as by activating the solid substrate or the probe with suitable chemical groups, such as aldehyde or amine groups or streptavidin (substrate) or amine or thiol groups or biotin (probe). In a particular embodiment, probes are fixed to the solid substrate by covalent bonding.

The array provided by the instant disclosure an be manufactured by any suitable method, e.g., ink-jet, syringe-solenoid, pin printing, microspotting, photolithography, etc.

In other aspect, the disclosure relates to a kit, hereinafter referred to as a kit of the disclosure, which comprises a set of probes for detecting and identifying the set of 85 genes shown in Table 2 and Table 3, wherein the set of probes comprises, or consists of, at least, one probe for each gene to be identified. The kit may also can contain one or more probes useful as hybridization controls. In an embodiment, the kit comprises of a plurality of probes hybridizing with specific sequence fragments of each nucleic acid (DNA, cRNA, etc.) corresponding to each one of the 85 genes disclosed in Tables 2 and 3. Preferably, the set of probes is deposited on a solid substrate (support) forming, for example, an array as that previously mentioned.

In a particular embodiment, the kit is useful for *in vitro* diagnosing NASH in a subject, or for early diagnosing NASH in a subject, or for determining the predisposition of a subject to develop NASH, or for determining the stage or severity of NASH in a subject, or for monitoring the effect of the therapy administered to said subject with said condition. Said aims can be achieved by quantifying the expression levels of said set of 85 genes shown in Tables 2 and 3 by conventional methods, e.g., by nucleic acid array hybridization technology.

In other aspect, the invention relates to an *in vitro* method for identifying and/or evaluating the efficacy of a potentially therapeutic agent against NASH, which comprises:
a) contacting a culture of liver cells with a test compound under the appropriate conditions and for the required period of time for them to interact;
b) determining the levels of the set of 85 genes shown in Table 2 and Table 3;
c) comparing said levels obtained in step b) to those of a control culture of liver cells lacking said test compound; and
d) selecting a test compound which causes (i) a decrease of the levels of genes shown in Table 2, and (ii) an increase in the levels of genes shown in Table 3 for further testing as a potential agent for the prophylactic and/or therapeutic treatment of NASH.

In a particular embodiment, the liver cells derive from an animal model of NASH, such as, for example, a MATIA-KO mouse, or from a subject who has been diagnosed with NASH. Practically any substance can be used as test compound in the working of the above method.

### EXAMPLE 1

### Identification of the genomic signature of human NASH

### Animal Experiment

Gene expression was determined in liver samples from male MATIA-KO homozygous and wild-type (WT) littermates fed with a standard diet. Liver samples were obtained fifteen-days, one-, three-, five- and eight-months after birth. Liver specimens were snap frozen for gene expression analysis and stored at -80°C. At least three independent liver samples were used for RNA isolation and measurement of gene expression for each age and condition. Gene expression profiles of liver samples from MATIA-KO and WT mice at 15 days, 1, 3, 5 and 8 months of age were obtained using the corresponding chip (see below).

### Patients

- Normal liver subjects: normal hepatic function with normal liver histology, n=6 (3 female, 3 male; mean age 57.6 years; range 23 - 79 years);
- NASH predisposed subjects: NASH grade 1 histologically diagnosed, n=9 (7 female, 2 male; mean age 41.1 years; range 24 - 61 years) [NASH grade 1 was histologically established (macrovesicular steatosis, lobular, portal inflammation and Mallory bodies) in the absence of other (viral, alcohol, metabolic) causes of NASH [Brunt EM, et al., 1999, Am. J. Gastroenterol. 94, 2467-2474]; and
- Steatosis diagnosed subjects: fatty acid accumulation without necrosis, histologically diagnosed, n=6 (4 female, 2 male; mean age 43.3 years; range 23 - 72 years).

Liver biopsies were divided into two parts, one was processed for routine histology and the other was immediately frozen in liquid nitrogen and stored at -80°C for subsequent RNA isolation and measurement of gene expression.

In order to establish a prognostic tool for designing NASH treatment regimens, expression patterns in NASH grade 1 specimens were assessed and correlated with clinical outcome.

### Tissue Processing/Method

RNA isolated from mice liver specimens (MATIA-KO and WT) and human liver specimens (NASH, steatosis and control) was reverse transcribed and resultant cDNA was used for *in vitro* transcriptional synthesis of fluorescently labelled nucleic acid probes according to manufacturer's instructions. Briefly, after column cleanup of the RNA (Qiagen Rneasy), cDNA was synthesized by using an oligo-dT primer (Superscript choice system; Life Technology) attached to a sequence of the T7 promoter region. This cDNA was then used to perform *in vitro* transcription by using a bacteriophage T7 RNA polymerase promoter and incorporating biotin-labeled nucleotides (IVT Labeling kit, Affymetrix). Labelled cRNA was purified; spectrophotometrically quantified, fragmented and separately hybridized to the corresponding expression array.

For mice liver tissue samples, each resultant tissue-derived probe was then separately hybridized to a single mouse expression array, namely, GeneChip Mouse Genome 430A 2.0 Array (Affymetrix), that contains over 22,600 probe sets representing transcripts and variants from over 14,000 well-characterized mouse genes that can be used to explore mechanisms behind biological and disease processes. Sequences used in the design of the array were selected from GenBank, dbEST, and RefSeq. The Sequence cluster was created from the UniGene database (Build 107, June 2002) and then refined by analysis and comparison with the publicly available draft assembly of the mouse genome from the Whitehead Institute Center for Genome Research (MSCG, April 2002). Oligonucleotide probes complementary to each corresponding sequence were synthesized *in situ* on the array. Eleven pairs of oligonucleotide probes were used to measure the level of transcription of each sequence represented on the GeneChip Mouse Genome 430A 2.0 Array.

For human liver tissue simples, each resultant tissue-derived probe was then separately hybridized to the GeneChip Human Genome U133A 2.0 Array (Affymetrix), a single array representing 14,500 well-characterized human genes that can be used to explore human biology and disease processes. It provides coverage of well-substantiated genes in the transcribed human genome on a single array. It analyzes the expression level of 18,400 transcripts and variants, including 14,500 well-characterized human genes. It is comprised of more than 22,000 probe sets and 500,000 distinct oligonucleotide features. All probe sets represented on the GeneChip Human Genome U133A Array are identically replicated on the GeneChip Human Genome U133A 2.0 Array. Sequences used in the design of the array were selected from GenBank®, dbEST, and RefSeq. The sequence clusters were created from the UniGene database (Build 133, April 20, 2001) and then were refined by analysis and comparison with a number of other publicly available databases including the Washington University EST trace repository and the University of California, Santa Cruz Golden-Path human genome database (April 2001 release).

In both cases, the arrays were washed, stained, scanned by using a confocal scanner (Affymetrix) and the hybridization images were analyzed by using the Genechips Operating Software (GCOS) Version 1.1 (Affymetrix) in order to generate a data matrix of named probes by quantitative expression level in each tissue.

In order to select a set of early gene markers for NASH, the expression data generated by the analysis of gene expression is analyzed to determine which genes in the different groups, for example KO versus WT mice, of liver tissues samples present a significantly different expression. Significant differences in gene expression levels were determined using the Affymetrix MAS 5.0 software. Signals for each entry were normalized. Final expression level is the difference from the mean expression of the signal for each gene measured in standard deviations. Two statistical filters to select gene markers of NASH in mice were used. Genes were firstly analyzed statistically using an ANOVA type I test (http://www.uv.es/~lejarza/anova/anova.html) and 3,508 genes passed this first test (p<0.025). As it is known, this test shows which genes behave coherently within each class. This test is useful to remove genes whose profile varies because of reasons other than age or type of mouse. The classes in which the genes were divided were 10 (WT 15 days, 1, 3, 5 and 8 months; and MAT1A-KO 15 days, 1, 3, 5 and 8 months). Genes were then statistically analyzed using a t-test (http://home.clara.net/sisa/t-thlp.htm) and 3,266 genes passed this second test (p<0.025). This test selects genes that have an average expression significantly different between WT and MAT1A-KO mice not taking into account the age of the mice. Matching these two sets of genes, it was identified a group of 1,496 common genes to both tests. These are genes that have different expression between WT and KO mice and behave coherently within each group.

In order to validate which of said mouse genes were also gene markers of NASH in humans, a microarray analysis of gene expression was carried out in order to determine which human genes in the different groups, for example, non-NASH and NASH liver tissue samples, present a significantly different expression, using the same process as described above. The set of human genes, presenting different expression levels between the two groups after the two statistical filters, corresponded to a set of homolog mouse genes. Therefore, gene expression profiles of liver samples from 6 subjects with normal liver function and 9 patients with NASH grade 1 using the Human Genome U133A 2.0 Array (Affymetrix) containing 54,675 entries were analyzed. Statistical analysis of these two sets of human samples using a t-test (p<0.05) identified a group of 4,272 discriminative human genes (4,679 Affymetrix probes) corresponding to 1,700 homolog mouse genes (2,556 Affymetrix mouse probes). Matching this set of 1,700 genes with the 1,496 discriminative genes identified above as gene markers of NASH in mouse, a set of 218 common gene markers of NASH to human and mouse was revealed.

Finally, in order to validate which of this set of genes are early/prognostic gene markers of NASH, gene expression profiles of liver tissues from 15 and 30 days old WT and MATIA-KO mice (representative of a very.early stage in NASH development) were examined. This comparison allows selecting genes that have an average expression significantly different between WT and KO mice early in the development of NASH. Statistical analysis of these two sets of samples, t-test with a p<0.025, was performed and a group of 1,346 discriminative genes was identified. Matching this set of genes with the 218 common NASH gene markers for human and mouse identified above revealed a set of 85 discriminative genes (Tables 2 and 3) that fulfill the following four criteria:
they are associated to a disease both in human and mouse,
they are early gene markers of the disease under study,
they remain differentially expressed during development of the disease, and
their variance is small during development of the disease.

As it has been previously mentioned, in order to analyse and compare gene expression patterns, the Affymetrix MAS 5.0 software was used, the signals being normalized for each entry and the final expression level being the difference from the mean expression of the signal for each gene measured in standard deviations. In addition, a nonlinear transformation (arcsinh) was applied to said values in order to diminish the effect of outliers. Further statistical analysis (ANOVA and t-test as above described) were used although any standard statistical package that can discriminate significant differences in expression may be used.

The set of genes, which has been termed as the genomic signature of NASH, was identified and classified according to their molecular function following the criteria of the Gene Ontology Consortium databases (http://www.geneontology.org/external2go/tigr2go) and Gene Atlas databases (http://www.citi2fr/GENATLAS/welcome.htlm) and other public sources using GARBAN (http://www.garban.org). Using this kind of genomic signature, groups can be divided into KOs mice with KOs mice and WT mice with WT mice, for example using this cluster of 85 genes associated to NASH it is possible to group MATIA-KOs with MATIA-KOs, WT mice with WT mice, NASH patients with NASH patients, and healthy controls with healthy controls.

Tables 2 and 3 (previously shown) identify 85 genes which accurately distinguish between patients with early-stage NASH and healthy controls, thus offering the prospect of early diagnosis of NASH, as well as the possibility of identifying patients having a certain risk for the development of NASH, which improves the chance to intervene with an effective treatment.

## Claims

1. A method for *in vitro* diagnosing non-alcoholic steatohepatitis (NASH) in a subject, or for early diagnosing NASH in a subject, or for determining the predisposition of a subject to develop NASH, or for determining the stage or severity of NASH in a subject, or for monitoring the effect of the therapy administered to said subject with said condition, which comprises:
a) quantifying the expression level of the set of 85 genes, shown in Table 2 and Table 3, in a liver tissue sample from said subject, and
b) comparing the expression levels of said genes to the expression level of the same genes of a control sample;
wherein an increase in the expression levels of the genes of Table 2 and a decrease in the expression level of the genes of Table 3, relative to the expression level of the same genes of the control sample, is indicative of NASH or indicative of predisposition of the subject to develop NASH.

2. Method according to claim 1, wherein the sample to be analysed is from a subject not previously diagnosed with NASH or from a subject who has been previously diagnosed with NASH, or from a subject receiving anti-NASH treatment, or from a subject who has received anti-NASH treatment previously.

3. Method according to claim 1, wherein the quantification of said gene expression level is carried out by solid phase nucleic acid microarray hybridization technology.

4. Use of the set of 85 genes, shown in Table 2 and Table 3, for *in vitro* diagnosing non-alcoholic steatohepatitis (NASH) in a subject, or for early diagnosing NASH in a subject, or for determining the predisposition of a subject to develop NASH, or for determining the stage or severity of NASH in a subject, or for monitoring the effect of the therapy administered to said subject with said condition.

5. A solid-phase nucleic acid array consisting of a set of probes useful for detecting and identifying all the genes shown in Tables 2 and 3, fixed to a solid substrate, together with one or more hybridization controls.

6. Use of a kit comprising a set of probes for detecting and identifying the set of 85 genes shown in Table 2 and Table 3, wherein the set of probes comprises, at least, one probe for each gene to be identified or a solid-phase nucleic acid array according to claim 5 for *in vitro* diagnosing non-alcoholic steatohepatitis (NASH) in a subject, or for early diagnosing NASH in a subject, or for determining the predisposition of a subject to develop NASH, or for determining the stage or severity of NASH in a subject, or for monitoring the effect of the therapy administered to said subject with said condition.

7. An *in vitro* method for identifying and/or evaluating the efficacy of a potentially therapeutic agent against NASH, which comprises:
a) contacting a culture of liver cells with a test compound under the appropriate conditions and for the required period of time for them to interact;
b) determining the level of gene expression of the set of 85 genes shown in Table 2 and Table 3;
c) comparing said level obtained in step b) to that of a control culture of liver cells lacking said test compound; and
d) selecting a test compound which causes (i) a decrease of the level of gene expression of all genes shown in Table 2, and (ii) an increase in the expression levels of all genes shown in Table 3 for further testing as a potential agent for the prophylactic and/or therapeutic treatment of NASH.

## Patentansprüche

1. Verfahren zur *in-vitro*-Diagnose von nicht-alkoholischer Steatohepatitis (NASH) bei einem Probanden oder zur Früherkennung von NASH bei einem Probanden oder zur Bestimmung der Prädisposition eines Probanden, NASH zu entwickeln, oder zur Bestimmung des Stadiums und Schweregrads von NASH bei einem Probanden oder zur Überwachung des Effekts der Therapie, die bei dem Probanden mit diesem Leiden durchgeführt wird, wobei das Verfahren:
a) die Quantifizierung des Expressionsniveaus der Gruppe von 85 Genen, die in Tabelle 2 und Tabelle 3 aufgeführt sind, in einer Lebergewebeprobe des Probanden und
b) den Vergleich der Expressionsniveaus dieser Gene mit dem Expressionsniveau derselben Gene in einer Kontrollprobe
umfasst und wobei eine Anstieg der Expressionsniveaus der Gene von Tabelle 2 und eine Abnahme des Expressionsniveaus der Gene von Tabelle 3 bezüglich des Expressionsniveaus derselben Gene in der Kontrollprobe NASH oder die Prädisposition des Probanden, NASH zu entwickeln, anzeigt.

2. Verfahren nach Anspruch 1, wobei die zu untersuchende Probe von einem Probanden, bei dem NASH bisher noch nicht diagnostiziert wurde, oder von einem Probanden, beim dem NASH bereits diagnostiziert wurde, oder von einem Probanden, der eine Anti-NASH-Behandlung erhält, oder von einem Probanden, der bereits eine Anti-NASH-Behandlung erhalten hat, stammt.

3. Verfahren nach Anspruch 1, wobei die Quantifizierung des Genexpressionslevels mittels Festphasen-Nukleinsäure-Mikroarray-Hybridisierungstechnologie durchgeführt wird.

4. Verwendung der Gruppe von 85 Genen von Tabelle 2 und Tabelle 3 zur *in-vitro-*Diagnose von nicht-alkoholischer Steatohepatitis (NASH) bei einem Probanden oder zur Früherkennung von NASH bei einem Probanden oder zur Bestimmung der Prädisposition eines Probanden, NASH zu entwickeln, oder zur Bestimmung des Stadiums und Schweregrads von NASH bei einem Probanden oder zur Überwachung des Effekts der Therapie, die bei dem Probanden mit diesem Leiden durchgeführt wird.

5. Festphasen-Nukleinsäure-Array, bestehend aus einer Reihe von Sonden, die zum Nachweis und zur Identifizierung aller Gene von Tabelle 2 und 3 verwendbar und an einem festen Substrat immobilisiert sind, und einer oder mehreren Hybridisierungskontrollen.

6. Verwendung eines Kits, umfassend eine Reihe von Sonden zum Nachweis und zur Identifizierung der Gruppe von 85 Genen, die in Tabelle 2 und Tabelle 3 aufgeführt sind, wobei die Reihe von Sonden mindestens eine Sonde für jedes zu identifizierende Gen umfasst, oder eines Festphasen-Nukleinsäure-Arrays nach Anspruch 5 zur *in-vitro*-Diagnose von nicht-alkoholischer Steatohepatitis (NASH) bei einem Probanden oder zur Früherkennung von NASH bei einem Probanden oder zur Bestimmung der Prädisposition eines Probanden, NASH zu entwickeln, oder zur Bestimmung des Stadiums und Schweregrads von NASH bei einem Probanden oder zur Überwachung des Effekts der Therapie, die bei dem Probanden mit diesem Leiden durchgeführt wird.

7. *In-vitro*-Verfahren zur Identifizierung und/oder Bewertung der Wirksamkeit eines potentiellen Wirkstoffs gegen NASH, welches umfasst:
a) das In-Kontakt-Bringen einer Leberzellkultur mit einer Testsubstanz unter den geeigneten Bedingungen und über den Zeitraum, die für ihre Wechselwirkung notwendig sind;
b) die Bestimmung des Genexpressionsniveaus der Gruppe von 85 Genen, die in Tabelle 2 und Tabelle 3 aufgeführt sind;
c) den Vergleich des in Punkt b) bestimmten Expressionsniveaus mit dem einer Kontrollkultur von Leberzellen, welche die Testsubstanz nicht enthält, und
d) die Auswahl einer Testsubstanz, welche (i) eine Abnahme des Genexpressionsniveaus aller Gene von Tabelle 2 und (ii) einen Anstieg der Genexpressionsniveaus aller Gene von Tabelle 3 verursacht, für die weitere Prüfung als potentieller Wirkstoff für die prophylaktische und/oder therapeutische Behandlung von NASH.

## Revendications

1. Procédé pour diagnostiquer, *in vitro,* une stéatohépatite non alcoolique (NASH) chez un sujet, ou pour diagnostiquer précocement une NASH chez un sujet, ou pour déterminer la prédisposition d'un sujet à développer une NASH, ou pour déterminer le stade ou la gravité d'une NASH chez un sujet, ou pour contrôler l'effet de la thérapie administrée à un sujet avec ladite affection, qui comprend :
a) la quantification du niveau d'expression du jeu de 85 gènes, illustrée dans le tableau 2 et dans le tableau 3, dans un échantillon de tissu de foie provenant du dit sujet, et
b) la comparaison des niveaux d'expression desdits gènes avec le degré d'expression des mêmes gènes d'un échantillon témoin ;
dans lequel un accroissement des niveaux d'expression des gènes mentionnés dans le tableau 2 et une diminution du niveau d'expression des gènes mentionnés dans le tableau 3, par rapport au niveau d'expression des mêmes gènes de l'échantillon témoin, sont indicatifs d'une NASH ou indicatifs d'une prédisposition du sujet à développer une NASH.

2. Procédé selon la revendication 1, dans lequel l'échantillon destiné à être analysé est prélevé d'un sujet auquel on n'a pas préalablement diagnostiqué une NASH, ou d'un sujet auquel on a préalablement diagnostiqué une NASH, ou d'un sujet recevant un traitement anti-NASH, ou d'un sujet ayant préalablement reçu un traitement anti-NASH.

3. Procédé selon la revendication 1, dans lequel la quantification dudit niveau d'expression de gènes est effectuée par une technologie d'hybridation de puce à acides nucléiques en phase solide.

4. Utilisation du jeu de 85 gènes, illustrée dans le tableau 2 et dans le tableau 3, pour diagnostiquer, *in vitro,* une stéatohépatite non alcoolique (NASH) chez un sujet, ou pour diagnostiquer précocement une NASH chez un sujet, ou pour déterminer la prédisposition d'un sujet à développer une NASH, ou pour déterminer le stade ou la gravité d'une NASH chez un sujet, ou pour contrôler l'effet de la thérapie administrée à un sujet avec ladite affection.

5. Puce à acides nucléiques en phase solide, consistant en un jeu de sondes utiles pour détecter et identifier tous les gènes illustrés dans les tableaux 2 et 3, liées à un substrat solide, ensemble avec un ou plusieurs témoins d'hybridation.

6. Utilisation d'un kit comprenant un jeu de sondes pour détecter et identifier le jeu de 85 gènes, illustrée dans le tableau 2 et le tableau 3, dans laquelle le jeu de sondes comprend au moins une sonde pour chaque gène destiné à être identifié, ou un réseau d'acides nucléiques en phase solide selon la revendication 5 pour diagnostiquer, *in vitro,* une stéatohépatite non alcoolique (NASH) chez un sujet, ou pour diagnostiquer précocement une NASH chez un sujet, ou pour déterminer la prédisposition d'un sujet à développer une NASH, ou pour déterminer le stade ou la gravité d'une NASH chez un sujet, ou pour contrôler l'effet de la thérapie administrée à un sujet avec ladite affection.

7. Procédé *in vitro* pour identifier un agent thérapeutique potentiel contre la NASH et/ou évaluer son efficacité, qui comprend :
a) la mise en contact d'une culture de cellules hépatiques avec un composé d'essai dans les conditions appropriées et pendant la période de temps requise pour que ceux-ci interagissent ;
b) la détermination du niveau d'expression des gènes du jeu de 85 gènes, illustrée dans le tableau 2 et dans le tableau 3 ;
c) la comparaison dudit niveau obtenu dans l'étape b) avec celui d'une culture témoin de cellules hépatiques, exempte dudit composé d'essai ; et
d) la sélection d'un composé d'essai qui provoque (i) une diminution du niveau d'expression de gène de tous les gènes mentionnés dans le tableau 2, et (ii) un accroissement des niveaux d'expression de tous les gènes mentionnés dans le tableau 3, afin de le tester ultérieurement en tant qu'agent potentiel pour le traitement prophylactique et/ou thérapeutique de la NASH.
